# DEMANDE DE BREVET EUROPEEN

(11) **EP 2 210 631 A2**
(43) Date de publication de la demande: **28.07.2010**
(21) Numéro de dépôt: 10162139.9
(22) Date de dépôt: 02.06.2005
(51) Int. Cl.: A61M 5/14

(54) **Poche de perfusion avec rincage intégré**

(30) Priorité: 02.06.2004 FR 0405919
(62) Demande divisionnaire de: 05826796.4
(71) Demandeur: Laboratoire Aguettant, 69007 Lyon (FR)
(72) Inventeur: Frezza, Pierre, 69390 Charly (FR)
(74) Mandataire: Maureau, Philippe

(57) **Abrégé**

La présente invention concerne une poche à usage médical pour perfusion d'un médicament par gravité, comportant 3 compartiments, l'un contenant un médicament sous forme d'une solution et les deux autres un soluté de rinçage et des moyens de séparation/communication des compartiments ne permettant au soluté de rinçage de pénétrer automatiquement dans le compartiment contenant le médicament qu'en fin de perfusion.

Le soluté de rinçage termine la perfusion donc rince la poche de médicament et la ligne de perfusion, permettant ainsi de supprimer les risques de contamination et la perte du médicament résiduel de la poche et de celui contenu dans la ligne de perfusion.

## Description

La présente invention concerne la perfusion de médicaments contenus dans une poche souple et s'écoulant dans les veines du patient par l'intermédiaire d'une ligne de perfusion sous l'action de la gravité, la poche étant suspendue au-dessus du patient.

Plus particulièrement, l'invention concerne une poche à usage médical pour perfusion d'un médicament par gravité, comportant au moins 2 compartiments, l'un contenant un médicament et l'autre un soluté de rinçage, et des moyens de séparation/communication permettant le rinçage automatique de la poche de médicament et de la ligne de perfusion.

L'invention propose de résoudre de manière simple et peu coûteuse, deux problèmes majeurs qui se posent lors de ce type de perfusion, à savoir :
- la perte d'une partie du médicament : La ligne de perfusion a un volume non négligeable, en particulier pour les poches de perfusion de faible taille. La quantité de médicament restant dans la ligne ajoutée à la quantité résiduelle contenue dans la poche n'est pas perfusée au patient qui, de ce fait, ne reçoit pas la posologie prescrite.
- les risques de contamination dus à la toxicité du médicament : Les médicaments perfusés peuvent être extrêmement toxiques ou allergisants (anticancéreux, antibiotiques, etc.) et induire des risques de contamination lorsque le personnel soignant effectue la purge d'air de la ligne de perfusion avant connexion du patient ou lors de la déconnexion de la ligne de perfusion.

La solution à ces deux problèmes consiste à effectuer un rinçage de la ligne en début et en fin de perfusion et un rinçage de la poche en fin de perfusion à l'aide d'un soluté inoffensif et bon marché (chlorure de sodium isotonique par exemple).

On connaît déjà des dispositifs permettant d'effectuer un rinçage au moins partiel en limitant les pertes en médicament et les risques pour le personnel soignant.

Le brevet US 5 242 392 décrit un système de perfusion comprenant une chambre pour un liquide de rinçage reliée à la tubulure pour perfusion entre la poche de médicament et le dispositif d'injection et placée au-dessous du niveau de la poche de médicament. Lorsque l'administration du médicament est terminée, la solution de rinçage rince de manière automatique la tubulure pour perfusion et le dispositif d'injection.

Le brevet FR 2 794 983 décrit un système de perfusion en circuit fermé comprenant au moins une poche de médicament et des moyens de distribution et de perfusion, associés à une poche de rinçage de manière à ce que le soluté de rinçage puisse passer dans les moyens de distribution et de perfusion, et en outre des moyens de sélection permettant le passage dans les moyens de distribution et de perfusion du médicament et/ou du soluté de rinçage.

Le brevet FR 2 306 711 décrit un dispositif de perfusion comportant au moins deux récipients à perfusion suspendus à des hauteurs différentes, dont les tubulures sont reliées par exemple à l'aide d'une pièce de raccordement en Y, et un dispositif de soupape à deux entrées dont le fonctionnement est lié à la différence de hauteur des récipients de perfusion qui entraîne une différence de hauteur des colonnes de liquide.

Les documents WO 92 11881, US 4 512 764, WO 95 09020, EP 0 790 064, US 4 623 334 et WO 03 077974 décrivent des systèmes de perfusion ou d'injection comportant des dispositifs permettant le rinçage de la ligne de perfusion ou du système d'injection.

Ces dispositifs n'apportent pas une solution satisfaisante aux problèmes de sécurité et d'efficacité des systèmes de perfusion :
- Si la poche contient de l'air, elle pourra être complètement vidée mais une partie plus ou moins importante de la ligne de perfusion contiendra de l'air et la ligne ne pourra pas être rincée car l'air contenu dans la ligne serait injecté au patient (risque d'embolie gazeuse). La présence de l'infirmière est donc requise avant la fin de la perfusion afin d'effectuer la manoeuvre de rinçage au moment exact où la poche est complètement vidée et avant qu'une partie de la ligne ne se remplisse d'air.
- Si la poche ne contient pas d'air, elle ne pourra pas être complètement vidée et le liquide résiduel contenu dans la poche ne sera pas injecté au patient. Dans ce cas, la ligne pourra être rincée, mais pas la poche.
- Dans tous les cas, l'intervention de l'infirmière est nécessaire, ce qui entraîne une charge de travail supplémentaire.

La présente invention propose d'améliorer la sécurité et l'efficacité des systèmes de perfusion grâce à un dispositif permettant de réaliser le rinçage de la ligne et de la poche en fin de perfusion de manière automatique, sans intervention de l'infirmière.

La présente invention concerne une poche à usage médical pour perfusion d'un médicament par gravité, comportant au moins 2 compartiments, l'un contenant un médicament sous forme d'une solution et l'autre (les autres) un soluté de rinçage et des moyens de séparation/communication des compartiments ne permettant au soluté de rinçage de pénétrer automatiquement dans le compartiment contenant le médicament qu'en fin de perfusion. Le soluté de rinçage termine la perfusion donc rince la poche de médicament et la ligne de perfusion, permettant ainsi de supprimer les risques de contamination et la perte du médicament résiduel de la poche et de celui contenu dans la ligne de perfusion.

Le principe de base de l'invention consiste à utiliser la dépression qui s'établit en fin de perfusion dans la poche de médicament sous l'action de la hauteur de colonne d'eau de la ligne de perfusion. On observe une augmentation progressive de cette dépression en fin de perfusion au fur et à mesure de l'aplatissement de la poche par effet «siphon». Cette dépression peut atteindre environ - 100 mb.

Le liquide de rinçage qui est à la pression atmosphérique est poussé vers le compartiment contenant le médicament qui lui est en dépression par rapport à la pression atmosphérique.

Les moyens de séparation/communication comprennent un dispositif ruptible entre le compartiment contenant le médicament et le compartiment (l'un des compartiments) contenant le soluté et un dispositif assurant la communication automatique entre le compartiment contenant le médicament et le compartiment contenant le soluté ou entre les deux compartiments contenant le soluté.

Le dispositif assurant la communication automatique entre les compartiments peut être un canal de communication situé au-dessus du niveau du liquide dans lesdits compartiments lorsque la poche est suspendue verticalement.

En variante, le dispositif assurant la communication automatique entre les compartiments peut être une valve à seuil de pression.

Le compartiment ou les compartiments contenant le soluté de rinçage peut (peuvent) comporter une zone rétrécie vers l'extrémité supérieure de la poche.

Les dessins annexés permettront d'illustrer plus en détail la présente invention.
La figure 1 représente une poche de perfusion à deux compartiments comportant un canal de communication.
La figure 2 représente une poche de perfusion à deux compartiments comportant une valve à seuil de pression.
La figure 3 est une coupe de la valve à seuil de pression de la figure 2 avec ruptible intégré à l'état «non-passant».
La figure 4 représente la même valve à l'état «passant».
La figure 5 représente une poche de perfusion à trois compartiments comportant un canal de communication.

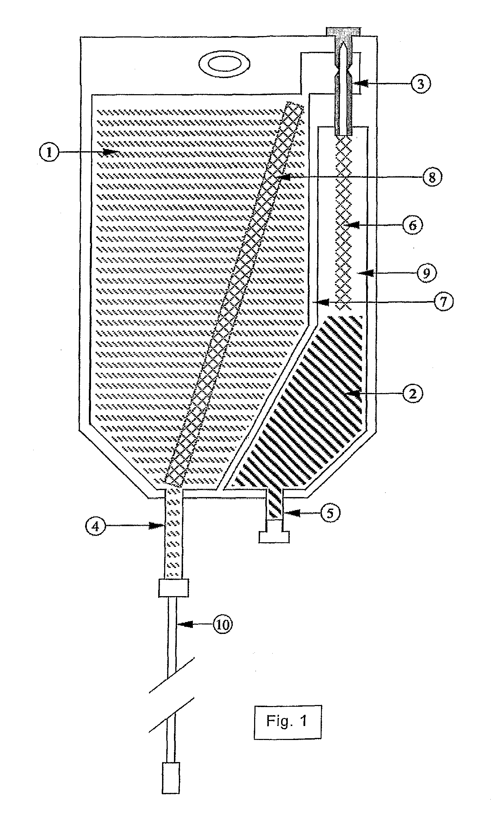

Selon un premier mode de réalisation de l'invention représenté sur la figure 1, on utilise une poche de perfusion souple séparée en deux compartiments (1) et (2) juxtaposés dont l'un (1) contient le médicament et l'autre (2) le soluté de rinçage. Le compartiment (1) comporte un accès (4) pour le remplissage de la poche et la connexion à la ligne de perfusion (10). Le compartiment (2) comporte une zone rétrécie (9) à l'extrémité supérieure de la poche et un accès (5) pour son remplissage. Les deux compartiments sont séparés par une paroi essentiellement verticale (7) dont la partie supérieure comporte un dispositif ruptible (3), conçu pour garantir l'étanchéité entre les deux compartiments lorsqu'il est intègre et permettre la communication entre les deux compartiments après sa rupture par l'infirmière par action mécanique sur l'extérieur de la poche. Ce dispositif est impérativement placé au-dessus du niveau des liquides lorsque la poche est suspendue.

Les compartiments (1) et (2) sont remplis sans adjonction d'air. Lesdits compartiments comportent des moyens (8) et (6) destinés à empêcher l'étanchéité totale du trajet d'écoulement du liquide lorsque la poche s'aplatit. Ces moyens (8) et (6) peuvent consister par exemple en un "grainage" de la surface d'une des faces de la poche ou en un canal thermoformé, qui facilitent l'écoulement.

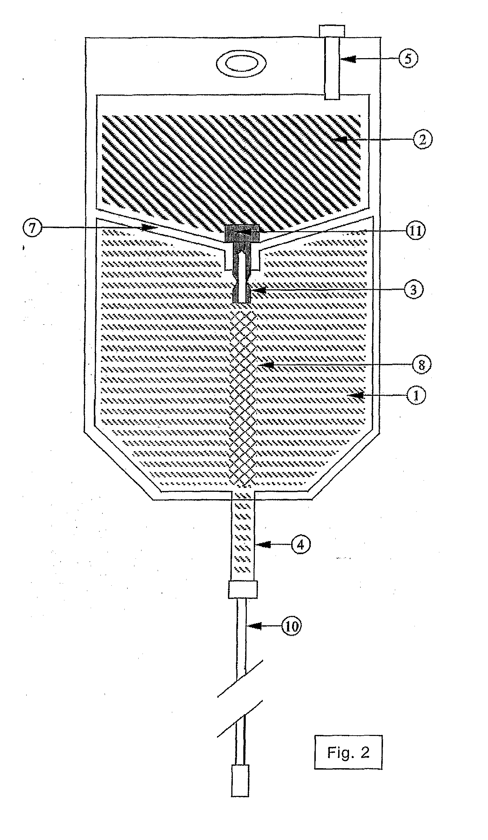

Selon un deuxième mode de réalisation de l'invention représenté sur la figure 2, on utilise une poche de perfusion souple séparée en deux compartiments (1) et (2) superposés. Les deux compartiments sont séparés par une paroi (7) essentiellement horizontale dont la partie centrale comporte un dispositif ruptible (3) rompu par l'infirmière. Dans ce mode de réalisation, l'écoulement du soluté de rinçage du compartiment (2) vers le compartiment (1) sera déclenché par une valve à seuil de pression (11) en fonction de la différence de pression entre les compartiments (1) et (2).

Le compartiment (1) comporte des moyens (8) destinés à empêcher l'étanchéité totale du trajet d'écoulement du liquide lorsque la poche s'aplatit. Ces moyens (8) facilitent l'écoulement entre l'orifice de communication des deux compartiments et l'accès (4) à la ligne de perfusion (10).

Selon une variante non représentée de la poche de la figure 1 ou 2, on utilise deux poches séparées au lieu d'une poche à deux compartiments.

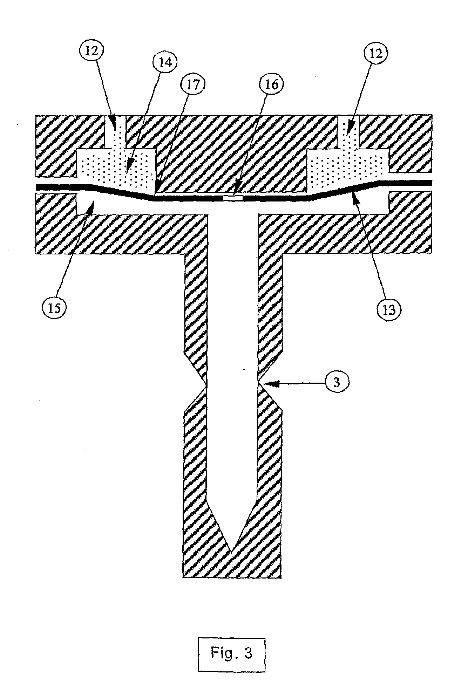

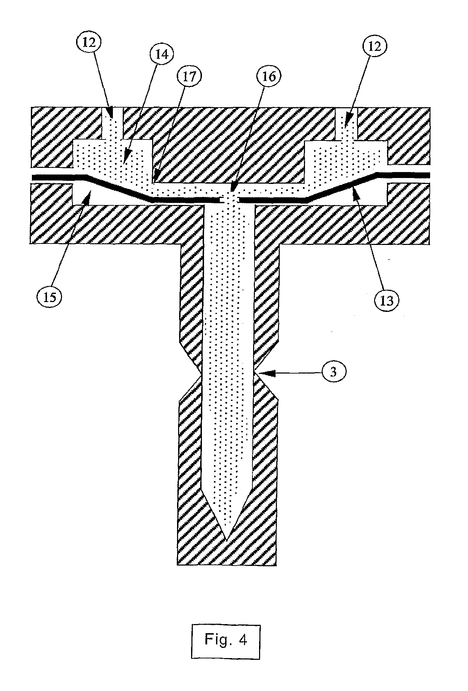

Les figures 3 et 4 représentent un exemple de la valve à seuil de pression utilisée dans la poche de la figure 2.

La valve comporte deux chambres (14) et (15) séparées par une membrane en élastomère (13). Comme représenté sur la figure 3, l'étanchéité entre les deux chambres est garantie par la prétension de la membrane (13) sur le jonc annulaire d'étanchéité (17). La chambre (14) est en communication avec le compartiment (2) contenant le soluté de rinçage par les ouvertures (12). La chambre (15) est en communication après rupture du dispositif ruptible (3) avec le compartiment (1) contenant le médicament.

Comme représenté sur la figure 4, lorsque la pression dans la chambre (15) devient négative par rapport à celle de la chambre (14), la membrane (13) se décolle du jonc annulaire d'étanchéité (17), permettant ainsi au liquide de rinçage contenu dans le compartiment (2) de s'écouler dans le compartiment (1) par l'intermédiaire de l'orifice (16).

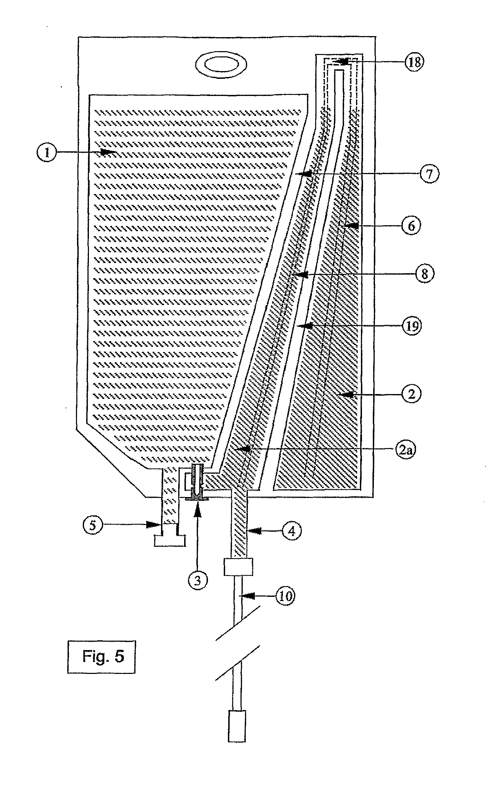

Selon un troisième mode de réalisation de l'invention représenté sur la figure 5, on utilise une poche de perfusion souple séparée en trois compartiments juxtaposés, qui permet un rinçage en début et en fin de perfusion. Le compartiment (1) contient le médicament et est séparé des compartiments (2) et (2a) contenant du soluté de rinçage par une paroi (7) essentiellement verticale dont la partie inférieure comporte un dispositif ruptible (3) rompu par l'infirmière.

Les compartiments (2) et (2a) sont séparés par une paroi (20) laissant un passage (18) au niveau supérieur de la poche, qui ne permet pas le transfert du contenu de (2) vers (2a) et/ou de (2a) vers (2) si la poche est suspendue verticalement et s'il n'existe pas de différence de pression entre lesdits compartiments. Le compartiment (2a) comporte un accès (4) à la ligne de perfusion (10). Le volume du compartiment (2a) est légèrement supérieur à celui d'une ligne de perfusion standard (environ 10 millilitres).

Les exemples ci-dessous illustrent la mise en oeuvre des poches de perfusion de l'invention.

### Exemple 1 : Poche à deux compartiments juxtaposés (figure 1)

Après installation de la perfusion, l'infirmière casse le dispositif ruptible (3) par action mécanique sur l'extérieur de la poche souple pour mettre en communication les compartiments (1) et (2) sans provoquer de transfert puisque la pression est identique dans les deux compartiments. Lorsque le compartiment (1) est pratiquement vide, ses parois se collabent. La pression dans le compartiment (1) devient alors négative par rapport à celle du compartiment (2) qui s'aplatit provoquant la montée de liquide de rinçage jusqu'au canal de communication entre les deux compartiments. Le liquide de rinçage se déverse alors dans le compartiment (1) puis dans la ligne de perfusion, rinçant de ce fait le compartiment (1) et la ligne (10) de la présence de médicament. Le patient aura donc reçu la totalité du médicament et l'infirmière manipulera une ligne de perfusion et une poche exempte de liquide toxique.

### Exemple 2 : Poche à deux compartiments superposés (figure 2)

On procède selon la méthode de l'exemple 1, sauf que c'est la valve à seuil de pression qui laisse passer le soluté de rinçage dans le compartiment (1), lorsque la pression dans ce compartiment devient négative par rapport à celle du compartiment (2).

### Exemple 3 : Poche à trois compartiments juxtaposés (figure 5)

Après connexion de la poche à la ligne de perfusion (10), la ligne de perfusion, remplie d'air est en communication avec le compartiment (2a) rempli de soluté de rinçage. L'infirmière effectue l'amorçage de la perfusion de manière habituelle avec le contenu du compartiment (2a). A la fin de cette opération, le compartiment (2a) est presque vide.

L'infirmière connecte la ligne au patient, puis casse le dispositif ruptible(3). Le compartiment (1) est alors en communication avec le compartiment (2a) et de ce fait avec la ligne de perfusion (10). Le médicament contenu dans (1) pénètre dans le compartiment (2a) jusqu'à équilibrage des hauteurs de liquide dans lesdits compartiments.

Lorsque les compartiments (1) et (2a) sont presque vides, les parois se collabent ce qui provoque l'aspiration du soluté de rinçage du compartiment (2) vers le compartiment (2a). Après équilibrage des niveaux entre les compartiments (1) et (2a), qui permet le rinçage du fond du compartiment, le soluté de rinçage s'écoule dans la ligne de perfusion (10).

La description et les figures sont une illustration de différents modes de réalisation de la présente invention. L'invention n'est cependant pas limitée aux modes de réalisation décrits et représentés mais elle couvre au contraire toutes les variantes.

## Revendications

1. Poche souple à usage médical pour une perfusion d'un médicament par gravité, comportant un accès (4) destiné à être connecté à une ligne de perfusion (10), comportant :
- au moins un premier compartiment (1), un second compartiment (2) et un troisième compartiment (2a), le premier compartiment (1) étant destiné à contenir un médicament sous forme d'une solution et les second et troisième compartiments (2, 2a) étant destinés à contenir un soluté de rinçage, le soluté de rinçage contenu dans le second compartiment (2) étant destiné à être aspiré vers le troisième compartiment (2a); et
- le premier compartiment (1) étant séparé des second et troisième compartiments (2, 2a) par une première paroi (7) essentiellement verticale ;
- les second et troisième compartiments (2, 2a) étant séparés par une deuxième paroi (19) laissant un passage (18) au niveau supérieur de la poche lorsqu'elle est suspendue verticalement ;
la poche étant **caractérisée en ce que** :
- le passage (18) est configuré pour empêcher le transfert du contenu du second compartiment (2) vers le troisième compartiment (2a) et/ou du contenu du troisième compartiment (2a) vers le second compartiment (2), lorsque la poche est suspendue verticalement et s'il n'existe pas de différence de pression entre lesdits compartiments (2, 2a) ;
- une partie inférieure de la première paroi (7) comporte un dispositif ruptible (3) conçu pour garantir l'étanchéité entre les premier et troisième compartiments (1 , 2a) avant sa rupture et permettre la communication entre les premier et troisième compartiments (1, 2a) après sa rupture,
- l'accès (4) est situé dans la partie inférieure du troisième compartiment (1), lorsque la poche est suspendue verticalement ;
- lesdits compartiments comportent des moyens destinés à empêcher l'étanchéité totale du trajet d'écoulement du soluté de rinçage lorsque la poche s'aplatit ;
et **en ce que**, la poche est conformée pour que, à partir d'une poche dont les premier et second compartiments (1, 2) ont été remplis respectivement par le médicament et le soluté de rinçage, une dépression augmente progressivement en fin de perfusion dans les premier et troisième compartiments (1, 2a), au fur et à mesure de l'aplatissement de la poche, par effet siphon, la dépression provoquant l'aspiration du soluté de rinçage du deuxième compartiment (2) vers le troisième compartiment (2a), lorsque les premier et troisième compartiments (1, 2a) sont presque vides.

2. Poche selon la revendication 1, **caractérisée en ce que** le second compartiment (2) comporte une zone rétrécie (9) à l'extrémité supérieure de la poche.

3. Poche selon la revendication 1 ou 2, **caractérisée en ce que** les moyens destinés à empêcher l'étanchéité totale du trajet d'écoulement du soluté de rinçage sont constitués par un grainage de la surface d'au moins une des faces de la poche.

4. Poche selon la revendication 1 ou 2, **caractérisée en ce que** les moyens destinés à empêcher l'étanchéité totale du trajet d'écoulement du soluté de rinçage sont constitués par un canal obtenu par thermoformage.
